# EUROPEAN PATENT SPECIFICATION

(11) **EP 3 112 102 B1**
(45) Date of publication and mention of the grant of the patent: **17.06.2020**
(21) Application number: 15174583.3
(22) Date of filing: 30.06.2015
(51) Int. Cl.: B26B 21/44

(54) **DISPOSABLE FLUID DISPENSING HAIR REMOVAL DEVICE**
WEGWERFBARE FLÜSSIGKEITSSPENDENDE HAARENTFERNUNGSVORRICHTUNG
DISPOSITIF D'ÉPILATION AVEC DISTRIBUTION DE FLUIDE JETABLE

(43) Date of publication of application: 04.01.2017
(73) Proprietor: The Gillette Company LLC, Boston, MA 02127 (US)
(72) Inventor: Shorey, Anthony William, Reading, Berkshire RG2 0QE (GB); Caton, Nicola Louise, Reading, Berkshire RG2 0QE (GB); Duke, Lottie Hannah, Bath, Somerset BA1 5DY (GB)
(74) Representative: P&G Patent Germany

(56) References cited:
- WO-A1-2009/019521
- WO-A2-2012/058216
- GB-A- 2 176 142
- US-A- 4 696 106
- US-A- 5 070 611
- US-A1- 2004 016 126
- US-B1- 7 007 389

## Description

### FIELD OF THE INVENTION

The invention relates to disposable fluid dispensing hair removal devices which enable a consumer to conduct a hair removal process without the need for additional system components which is low cost and simple to manufacture and convenient to use, particularly in the absence of a source of water and or outside of a bathroom environment.

### BACKGROUND OF THE INVENTION

Reusable hair removal devices which are also capable of dispensing a fluid are known in the art. Such devices typically include one or more fluid dispensing orifices through which the fluid is dispensed via the razor cartridge during the shaving process. The fluid is contained with a replaceable reservoir located in the handle and is activated by a push button. Such devices are complex to manufacture requiring many parts and are designed to enable the replacement of both the razor cartridge and fluid reservoir. For example see US2013/0145626, US 2013/0145625, US2013/0145601, US5070611 and WO2011/130372. Moreover such devices are typically large and cumbersome and not intended to be transported and used "on the go".

Disposable hair removal devices whereby the razor cartridge cannot be replaced, are also well known in the art. These devices are typically slightly less complex versions of reusable devices, for example by the removal of a pivot between the cartridge and handle, to thereby simplify and reduce the costs of manufacturing. Nevertheless, these devices are still considerably complex to manufacture.

Hence, there is still a need to provide a disposable hair removal device which also enables the dispensation of a fluid and thereby negates the need for a separate fluid container whilst reducing the complexity of the device and number of components thereof, to reduce the cost of manufacture. This is particularly desirable for hair removal processes which take place away from the consumers' home bathroom facilities such as whilst traveling or "on the go" and or in the absence of a convenient water supply. There is also a need to provide a device which is easy to effectively use for all body areas and in particular for specific areas and or touch up hair removal applications.

Attempts have been described in the art to provide disposable hair removal devices which also dispense a fluid. For example EP427889A describes a disposable razor with detachable gel packets secured to the razor handle whereby the packets can removed from the razor, opened and the contents applied to the skin prior or after the shaving process. This device is not particularly convenient for the consumer as the packets require removal from the device and separate independent application of the fluid. DE 102011117590 describes a disposable razor comprising a removable container for a shaving gel. The shaving gel container is placed on the razor handle and the end is placed on the razor head. Upon applying pressure to the perforations on the container the container is opened to release the razor gel. A similar embodiment is described in WO2009/019521.

US2004/0016126 describes a manually adjustable hair removal and skin lubrication device. The device has a U shaped body which can be manipulated to expel lubricant from the internal reservoir independently or simultaneously during the hair removal process. This device however requires a significant amount of manual dexterity in order to select the desired usage configuration and in particular, to consistently maintain the desired configuration in order to simultaneously dispense the lubricant and control the razor cartridge during the entire shaving process. The consumer is therefore required to continuously check the configuration and this is particularly inconvenient as it results in an interruption of the shaving process and is impractical when shaving more inaccessible body areas. Moreover, the lubricant and wicking device are not sealed prior to use and thereby are liable to inadvertent spillage and or contamination.

Despite the availability of fluid dispensing razors, many consumers however still prefer to apply a skin preparation treatment onto the skin prior to the shaving process even if using a liquid dispensing razor. Liquid dispensing razors typically dispense the liquid directly below or above the razor blades or from within the razor cartridge through the razor blades. Consequently, the consumer may not be able to visibly confirm that the liquid has been dispensed on the skin surface to be shaved, as the dispensing applicator is obscured from view by the shaver head. Moreover the consumer also cannot easily or correctly apply the blades to specific areas due to the restricted visibility. The use of the pre-shaving preparation reassures and confirms to the consumer that there is complete and thorough coverage of the skin with the skin preparation prior to shaving. Moreover, the skin is hydrated and lubricated prior to shaving which improves the shaving experience. Similarly, the removal of the composition from the skin following the shave provides an indication to the consumer as to which areas have been shaved. There is thus a need to provide a device wherein the hair removal and fluid application processes can be conducted d independently of one another such that the respective size and orientation of the hair removal and fluid applicator can be selected to facilitate their respective performance without affecting the performance of the other.

US1985132, US3492723, US5274922, US5819413 and WO2010/100634 describe disposable razors wherein the handle and or cartridge may be composed of cardboard. Such devices have long been proposed in the art as a means to provide cheap disposable razors. However they suffer from the disadvantage of retaining structural integrity due to water contact during shaving and thus commercially available products both reusable and disposable are typically provided with a plastic handle. The development of liquid dispensing razors which store and deliver liquid shaving compositions has further only cemented the use of plastic handles in the industry.

There is still a desire however to reduce the use of plastic materials or components particularly in disposable consumer items and or to utilize sustainable or renewable resources therefore. However biodegradable plastics or recycled plastics have been found not to provide the desired level of performance or functionality in part due to the variability in the quality of material and still require the use of expensive tooling and or molding equipment to form the desired ergonomic shapes.

In order to ensure a comfortable shave and enable the razor to move along the contours of the face for example razors are typically provided with a pivot point allowing movement of the razor cartridge relative to the handle. Such movement about the pivot point is limited in a single direction to ensure control of the device. Disposable razors on the other hand are typically not provided with such a pivot and the cartridge is in a fixed position relative to handle to reduce complexity and cost. Similarly in order to enable the user to provide a neat finish to side burns and moustaches; to easily shave difficult to access areas such as around the nose; and or in order to facilitate quick touch up shaving, razors are often provided with an additional trimmer blade. However, again disposable razors are typically not provided with an additional trimmer blade and thus these desirable shaving tasks cannot be readily achieved.

Consequently, there still exists a need to provide a cost effective, disposable fluid dispensing hair removal device which enables the application of a pre shave or post shave composition in a simple and convenient manner which is easy to use and does not require any manual dexterity and which can be used for all body areas and is suitable for use away from home and "on the go". There is also a need to provide a device which does not require expensive tooling or molding equipment. There is also a need to provide a device which readily follows the contours of the skin surface and also enables a neat finish to side burns and the like and enables effective shaving at difficult areas to access on the face and other body areas.

It has now been surprisingly found that disposable hair removal and fluid dispensing devices which are cheap and easy to manufacture, and which not requiring expensive tooling equipment, but deliver the desired level of functionality of the consumer products can be provided by a device having a hair removal means and fluid dispensing means located at the proximal and distal ends of the device respectively.

### SUMMARY OF THE INVENTION

The present invention relates to a hair removal and fluid application device according to the features as defined in claim 1.

In a further aspect the invention relates to a method to dispense fluid and or remove hair comprising the steps of contacting a skin surface with the hair removal means and or fluid dispensing means of a device (1) of the invention, preferably in the absence of a source of water.

### BRIEF DESCRIPTION OF THE DRAWINGS

Figures 1a and 1b shows perspective front and rear views of a first embodiment of the invention.
Figures 2a and 2b shows cross section views of second and third embodiments of the invention.
Figures 3a and 3b show a cross sectional and exploded view of a fourth embodiment of the invention.
Figures 4a, 4b and 4c; figures 5a and 5b and figures 6a and 6b show cross section views of fifth, sixth and seventh embodiments of the opening means of the invention respectively.
Figures 7a, 7b and 7c show a cross section and exploded cross section view of an eighth embodiment of the invention.
Figure 8a, 8b, and 8c show a cross section and exploded cross section view of a ninth embodiment of the invention.

### DETAILED DESCRIPTION OF THE INVENTION

According to the invention the hair removal and fluid application device (1) comprises a hair removal means (2), a fluid dispensing means (3), a handle (10) and a fluid reservoir contained therein, wherein said fluid dispensing means (3) is attached to the distal end of said handle and said hair removal means (2) is attached to the proximal end of said handle.

### Hair removal means

Any hair removal means (2) known in the art may be used herein, such as single or multiple, i.e. at least two blades, or three blades and optionally an associated razor housing or cartridge there for; foil, scraper or mesh. Preferably, the hair removal means comprises a guard and a cap with at least one blade located in-between the cap and guard. More preferably, the hair removal means (2) comprises a razor cartridge having a housing and a cap and a guard located on the housing and at least one blade(s) positioned between the cap and guard. Embodiments of this type having a single blade cartridge are particularly beneficial as they allow shaving debris such as hair, skin and shaving composition to readily pass through the cartridge and thereby prevent clogging.

### Fluid dispensing means

Suitable fluid dispensing means (3) include any material capable of dispensing the fluid upon application of the means against a user's skin. The rate of dispensing can be readily controlled by the consumer by regulating the amount of pressure applied to the dispensing means against the user's skin.

Suitable materials include foams, including open and closed cell foams, wovens, nonwovens, pressed porous fibres such as felt, single or multiple perforated or apertured films, rigid or semi rigid molded plastic and combinations thereof. The fluid dispensing means may comprise a single layer or multiple layers of material which may be the same material or different. Such layered embodiments may be layered vertically on top of one another whereby each layer extends towards the surface facing the skin contacting surface or layered adjacent one another each layer directly facing the skin contacting surface.

Preferably, the hair removal means (3) is provided by a foam, more preferably an open celled foam or a felt. Suitable materials include natural sponge, cellulose, polyethylene, polyurethane, silicone and other synthetic foam or felt materials known in the art and commercially available. Foam materials are particularly advantageous as they may function to both retain, dispense and spread the fluid dispensed onto the skin, thereby enabling a more controlled application of the fluid onto the user's skin by the consumer.

In one preferred embodiment, the fluid dispensing means is selected such that it may also function as a post hair removal debris collector so that it will act to collect at least some of the hair and or fluid remaining on the skin after the hair removal process. Suitable materials for such embodiments include foams preferably open celled cellulose foams. Alternatively a combination of materials may be utilized in order to provide both fluid dispensing and debris collection. Suitable combinations include for example open and closed cell foams, or a foam and non woven sheet material composite.

The fluid dispensing means preferably has a skin contacting surface area of from about 0.8cm² to about 35cm², preferably from about 5cm² to about 15cm^{2,} more preferably from about 8cm² to about 10cm².

The fluid dispensing means may be provided with at least one aperture or opening or channel (9) in fluid communication with the reservoir as discussed hereinafter.

### Handle

The hair removal and fluid application device (1) further comprises a handle (4). The handle enables a user to both hold and control the device and to securely locate and position the hair removal means and the fluid dispensing means in predetermined positions relative to one another Furthermore the handle contains the fluid reservoir (10). The handle (4) has a proximal end and a distal end and may have a major front and back surface. The hair removal means (2) is positioned at the proximal end of the handle and the fluid application means (3) is positioned at the distal end of the handle (4). In one embodiment the hair removal means (2) and fluid dispensing means (3) may be located on the front and back surfaces (7,8) respectively or they may be both positioned on the front or back surface. In another embodiment the fluid dispensing means is e located at the terminal end of the distal end of the handle (4) and may be positioned substantially perpendicular to the hair removal means (2). In another embodiment the hair removal means (2) and fluid dispensing means (3) each have a respective skin contacting surface, wherein the skin contacting surface of the hair removal means is positioned substantially in the opposing direction, to the skin contacting surface of the fluid dispensing means. In one embodiment the hair removal means (2) may be positioned at an angle to the handle (4) and or vertical plane of the device (1). The location of the hair removal means (2) and the fluid dispensing means (3) on the proximal and distal ends respectively enables the hair removal means and the fluid dispensing means to be contained on a single device but to be used independently from one another without any interference. This thereby enables the consumer to effectively apply the fluid composition to the desired body surfaces before or after use of the hair removal device. This is particularly advantageous for spot applications or side burns and beard trimming. The hair removal means (2) and the fluid dispensing means (3) may be positioned such that they are substantially aligned about a horizontal plane. Alternatively, the hair removal means and the fluid dispensing means may be offset. The handle (4) typically extends from the proximal end comprising the hair removal means (2) towards the distal end comprising the fluid dispensing means (3). This is in order to provide a portion of the handle which can be readily held and controlled by the consumer.

The fluid dispensing means (3) and hair removal means (2) are preferably independently attached to the respective distal and proximal ends of the handle (4) of the device (1). Suitable means include adhesives, ultrasonic welding, double sided tape, rivets, clips or other mechanical means and combinations thereof. The attachment of the fluid dispensing means and the hair removal means may be permanent or releasable to enable replacement thereof.

In one embodiment, the fluid dispensing means (3) and or hair removal means (2) independently extend along at least a portion, preferably at least 50% more preferably at least 75%, even more preferably at least 90% of the width of the proximal and distal ends respectively. In another embodiment the fluid dispensing means (3) and or hair removal means (2) independently extend along at least 95 %, preferably at least 99% of the width of the proximal and distal ends respectively.

The handle (4) preferably provides sufficient rigidity to the device to enable the consumer to hold the device and apply and dispense the fluid onto the skin or apply the hair removal device onto the skin. The handle may also preferably provide a degree of flexibility in order to facility the expulsion of the fluid from the reservoir contained within the handle by the consumer upon application of pressure. The handle is typically resilient and will resume its configuration upon removal of pressure.

Both the hair removal and fluid application means have an associated skin contacting surface. In order to maximise performance, it is desirable that in use the hair removal and fluid application means achieve maximum contact with the respective skin contacting surface. This requires that the hair removal means and fluid application means generally follow the contours of the skin surface, where they are used, e.g. leg, chest, and face. With conventional reusable shaving devices this is achieved by connecting the shaving head or cartridge to the body or handle of the device which held by the user using a hinge or bearing that pivots around an axis defined by the hinge or bearing. Such devices whilst effective increase cost and complexity and are generally less suitable for disposable devices.

Whilst not being bound by theory, it is believed that, in use, the disposable hair removal and fluid application device as described herein follows the contours of the skin surface by preferably enabling a deflecting motion of the device via the handle itself with respect to the at rest position (i.e. its position in the absence of the application of any external forces to the device, such as when the device is resting on a horizontal surface). The deflection motion of the device may occur in a portion of the proximal end or distal end of the handle and or device, preferably along the entire length of the handle between the proximal end and the distal end where it is held by the user. In contrast to conventional devices, the handle of the device described herein enables deflection in two opposing directions from its at rest position, depending on the orientation in which the hair removal and fluid application device is held and which of the two means are in use by the consumer. The deflection occurs in response to the user holding the device, and exerting a force onto the entire device when pushing it against the skin surface to apply either a fluid or remove hair which results in the device adopting the in use hair removal deflected position and or the in use fluid dispensing in use deflected position. The typical average mean load exerted by a user is from about 120g to about 200g.

The in use deflected positions may be provided by the selection of the material of the handle and or the configuration of the handle and or by the provision of fold lines on the handle, as discussed hereinafter, but preferably not a conventional pivot or hinge connecting the hair removal means and fluid dispensing means to the support.

In one embodiment the hair removal and fluid dispensing device has a vertical plane of symmetry. The at rest and in use deflected positions of the device and or handle may be determined with respect to the vertical plane of symmetry.

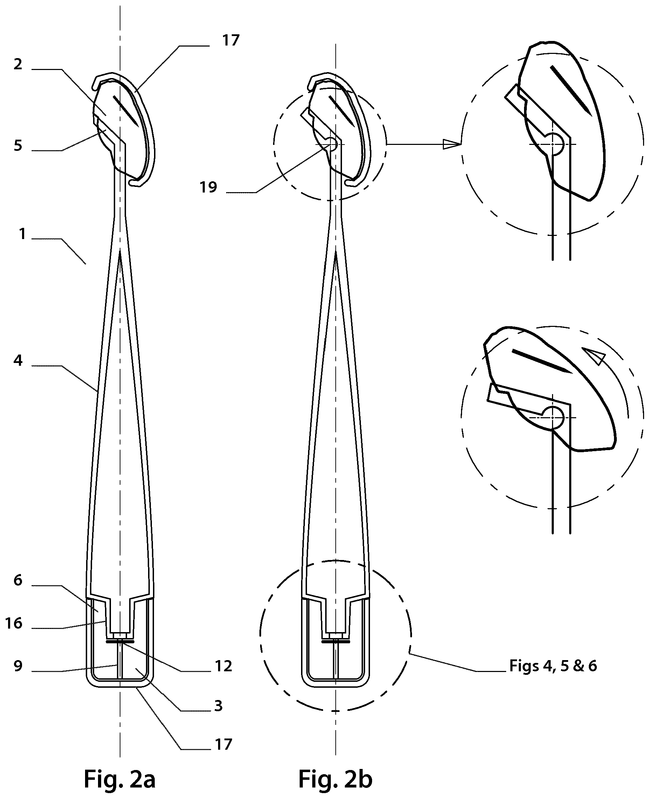

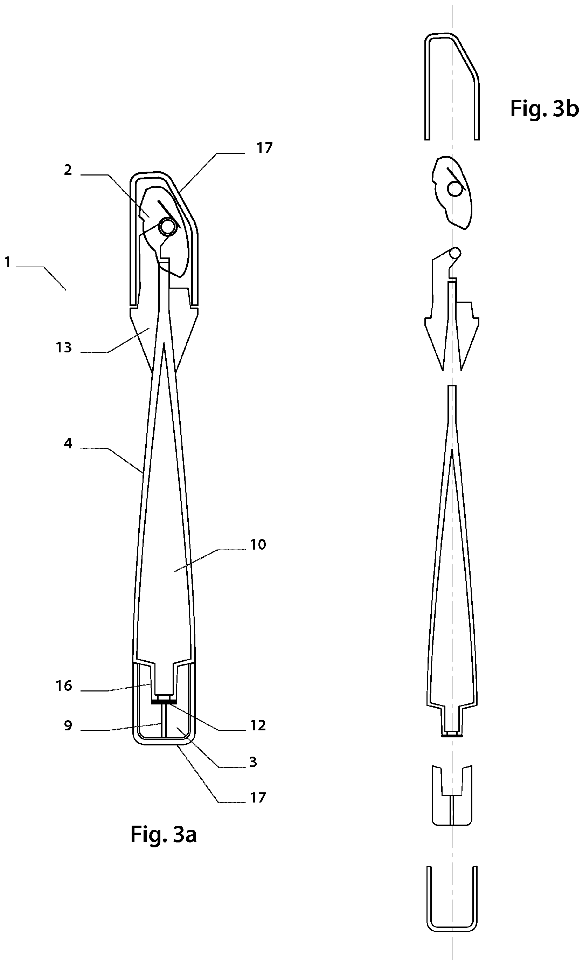

In an alternative embodiment of the invention the proximal end of the handle may be provided with a chassis (13) with a pivot to enable a pivot motion of the hair removal means. In another embodiment the hair removal means may be attached to the proximal end of the handle which is inclined at an angle or provided with a living hinge (19) in order to provide a degree of pivot motion to the hair removal means as shown in figures 2 and 3.

The handle may be formed from any suitable material include natural and synthetically derived materials and combinations thereof including polymers, such as plastic, both rigid and semi rigid such as polystyrene, polypropylene, which may be extruded, blow molded, injection molded, 3D printed, cellulose such as cardboard, paperboard, carton board and carrier board; nylon, rayon, cotton and combinations thereof. The handle may be in the form of a scaffold, cage, mesh, lattice or skeleton configuration, or in tubular form or combinations thereof optionally having openings therein. In one embodiment, the handle is substantially rigid. This may be of advantage for use in difficult to access areas or to provide a neat finish to side burn and beards for example.

According to the invention the handle (4) may be formed from carton board, preferably carrier board. The handle may be a single or multiple boards and or may be in the form of a scaffold, cage, mesh, lattice or skeleton configuration, or combinations thereof optionally having openings therein. The handle may be formed from any suitable cardboard, carrier board, carton board, paper board or liquid packaging board. Such terms are used interchangeably herein. Such boards are typically produced from cellulose fibres such as wood or plant based pulp sources included recovered fibres or waste paper. The carton board may be single or multiply. The carton board may contain pigment coating such as clay, calcium carbonate and titanium dioxide and or may contain adhesives and or binders such as styrene butadiene. The handle may preferably be surface treated on at least one major front and or rear surface with a suitable water repellant material such as a wax or polymer(s) to improve consumer handling particularly in the presence of water and to improve the tactile feel or grip of the handle and to more readily enable printing.

The material may have at least a portion which is corrugated. In one embodiment the carton board may be laminated to further improve wet strength. The handle may be formed from a single piece of material or from two or more pieces of material which are joined together using known means such as adhesive.

Whilst not being bound by theory the use of carton board for the handle provides a number of advantages. Carton board can be readily incorporated into a manufacturing process without the need for expensive tooling and is readily cut to the desired shape. Moreover as discussed hereinafter the desired fold lines or score lines are also readily produced in carton board. Carton board further provides a desirable thickness to strength ratio and density to provide a strong but lightweight handle. Consequently, the carton board handle can provide the device with the desired degree of flexibility for improved consumer usage experience whilst maintaining sufficient rigidity so that the consumer can exert the desired force associated with the hair removal or fluid application process as exemplified hereinafter. The carton board for use herein may have basis weight of at least about 200g/m², preferably at least about 225g/m², more preferably at least about 250g/m². The carton board may have a thickness of from about 0.25mm, preferably from about 0.30mm, more preferably from about 0.35mm and preferably less than 1.5mm.

The handle may take any shape or configuration provided it is suitable to be held by the consumer and can secure the location of the hair removal means and the fluid dispensing. Suitable configurations include substantially U shape, V shape, diamond shape, S shape substantially flat, tubular, curved shape and combinations thereof. The shape should preferably be ergonomic and enable easy handling by the consumer. Such configuration s may be provided by using known manufacturing techniques such as injection or blow molding optionally with the incorporation of living hinges (19) therein

At least a portion of the handle may be provided within the fluid reservoir (10) or, integral with the reservoir. Alternatively the handle may be provided external to the reservoir. In one embodiment the handle is external to the reservoir and partially, preferably substantially encloses the reservoir either on the major front and or rear surface. Such embodiments provide protection of the fluid reservoir from accidental rupture and enable the consumer to exert pressure onto the reservoir to expel the fluid contained therein via the handle.

In one embodiment the handle may be provided with at least one, preferably at least two predetermined fold lines (deformation lines) and or score line, typically positioned extending longitudinally from the proximal end to the distal end. The fold lines are preferably formed so as to enable a flat handle to form a 3D shape and contain the fluid reservoir enabling the consumer to readily hold the device Alternatively, the fold line(s) may provide further structure to a preformed 3D handle and or delineate or separate the hair removal means from the fluid dispensing means.

In another embodiment the handle may be provided with predetermined fold lines to provide a degree of pivot motion preferably between the handle and the hair removal device and or fluid dispensing means without the need for a complex pivot unit. In another embodiment the handle encloses the reservoir whilst enabling the consumer to visually observe the amount of fluid within the reservoir.

According to one embodiment of the invention the distal end of the handle has a skin contacting surface which is substantially at right angles to the front and or rear surface of the handle. The fluid dispensing means may extend from said skin contacting surface of the distal end of the handle as shown in the figures.

Alternatively the skin contacting surface of the distal end of the handle may form part of the front or rear surface of the handle. Similarly, the proximal end of the handle has a skin contacting surface which may be substantially perpendicular to the front and or rear surface of the handle or the skin contacting surface of the proximal end may form part of the front or rear surface of the handle.

### Reservoir

The device further comprises at least one sealed fluid reservoir (10), preferably a flexible reservoir more preferably contained within the handle (4). The reservoir (10) may extend from the distal end towards the proximal end of the handle (4) and is suitable to contain a fluid, paste or gel. The reservoir (10) of the invention may be any suitable reservoir to contain fluids. Preferably the reservoir is a flexible reservoir for containing fluids which facilitates the expulsion of the fluid from the reservoir upon the application of pressure by the user.

The reservoir typically has major front and back surfaces and at least one side edge, preferably the reservoir has two side edges and a top and a bottom edge. The edges may define the perimeter of the reservoir (10). The edges are preferably linear but may exhibit a degree of curvature for example at the respective corners. The fluid reservoir has a main body portion which contains the fluid. The at least one sealed edge, is preferably located on the top edge of the reservoir (10).

The fluid reservoir may be provided from any material or combination of materials suitable to contain a fluid i.e. liquid impervious materials or composites. In one embodiment the reservoir is formed from a polymeric film such as plastic films, and or laminated plastic films or composite materials such as for example: PET/VMPE, PET/Foil/PE (preferably metal foils for example aluminum), PET/LLDPE, PET/PE-EVOH-PE, or SURLYN™ or other commercially available materials which are preferably capable of being sealing, preferably by heat sealing techniques. The laminate films may be formed by any method known in the art such as heat, pressure and or adhesive. The material may be selected depending on the capacity of the reservoir and the density and the volume of the fluid to be contained therein and the strength and flexibility required for the particular application. The material may be transparent or opaque; the latter may have particular application to prevent fluid degradation. The outer surface of the reservoir or at least a portion thereof such as the major front or rear surface(s) or portion thereof, may be coated with an additional material to provide a consumer preferred tactile surface such as a woven, non woven, and or polymers such as silicone and rubber. In addition the outer surface of the reservoir may be provided with indicia to communicate to the consumer information such as the contents of the reservoir, usage instructions, recommended handling position to hold and dispense the fluid.

The reservoir (10) may be formed from a single sheet of material, which is folded and sealed, preferably heat sealed, at the top and bottom end edges and one side edge to form the reservoir (10). Optionally the second may be sealed to form a perimeter seal. In another embodiment, the reservoir may be formed from at least two sheets of material sealed along all the top, bottom, and side edges to form the reservoir. The edges of the reservoir are preferably substantially linear but may be partially curved. The reservoir may be sealed along all of its perimeter edges. Alternatively the reservoir may be formed by extrusion or blow molding techniques and may comprise one single sealed edge and no additional perimeter edge seals. The reservoir may be of any shape but is typically substantially rectangular, square, oval or circular, preferably substantially rectangular.

The reservoir (10) may comprise one or multiple i.e. two or more separate fluid compartments to enable different compositions to be applied and or to enable multiple applications of the same or similar fluid composition(s). Each separate fluid compartment will preferably have an opening means or tab associated with the reservoir as described hereinafter. The multiple compartments may be provided by forming a reservoir having one compartment which is divided into 2 compartments by the provision of an additional seal.

In certain embodiments the fluid reservoir may be attached to the handle (4) at least on a portion of one of the internal or external surfaces thereof. Any suitable means to attach the reservoir may be used such as adhesives, ultrasonic welding, double sided tape, rivets, clips or other mechanical means and combinations thereof.

In one embodiment, the fluid dispensing means may also function as the fluid reservoir. This is achieved by the selection of materials for the fluid dispensing means such as felt which is, capable of providing the required fluid capacity and releasing the fluid only upon demand. Such materials may additionally be in liquid communication with a fluid reservoir.

In another embodiment, the handle may be provided with a hollow cavity which functions as the fluid reservoir without the need for a separate fluid reservoir component as shown for example in figures 2a and 2b. In such embodiments, in order to contain the fluid and to enable the consumer to hold the reservoir in order to use the hair removal means and or fluid dispensing means in a controlled manner across all skin surfaces in both wet and dry environments, the hollow handle (10) may be provided from any material or combination of materials suitable to contain a fluid i.e. liquid impervious materials or composites and which is flexible, and preferably resilient provided as a single component. This enables the handle to provide the required both sufficient stiffness whilst retaining sufficient flexibility to be held securely by a user to utilize the device in a controlled manner without the need for a pump or push button activator or complex multicomponent parts. In one embodiment, the handle is formed from a polymer such as HDPE, MDPE, LDPE, PP, PET, PS, PC, PVC, PE or mixtures thereof, or other commercially available materials which are preferably capable of being blow molded. The material may be selected depending on the internal capacity of the handle and the density and the volume of the fluid to be contained therein and the strength and flexibility required for the particular application.

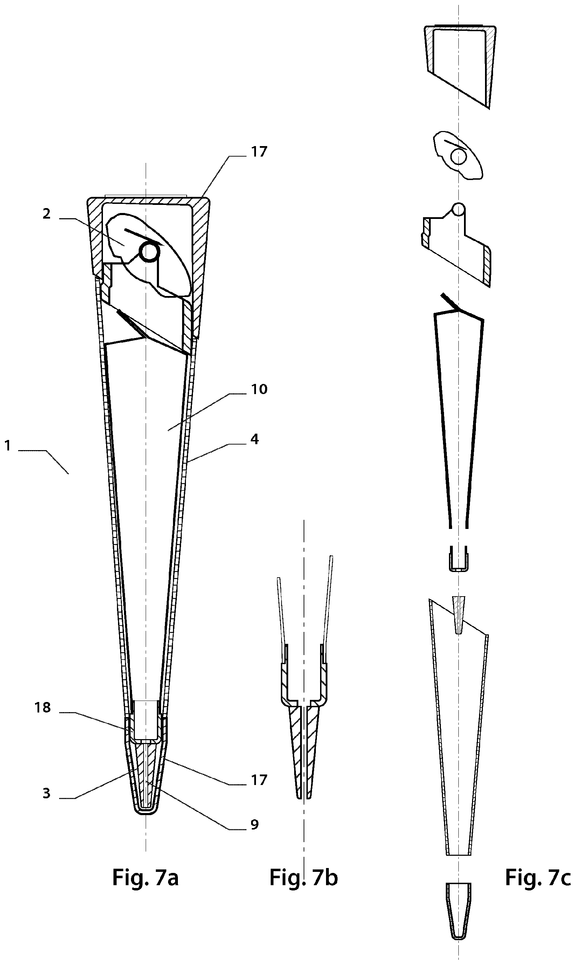

In one embodiment the distal end of the handle or reservoir may be provided with a plug insert which has an aperture or channel through to a felt nib as shown in figure 7.

### Capacity

The capacity of the fluid reservoir is selected dependent upon the end use and intended usage regime, in other words whether it is intended for single use or multiple use. For beauty and grooming applications, the fluid reservoir may have a capacity of from about 1ml to 500ml, preferably from about 1ml to 100ml, more preferably from 1ml to 15ml or from 10ml to 25ml. The fluid reservoir is typically filled to at least about 75%, preferably at least about 80% capacity to prevent inadvertent spillage upon opening.

### Opening means

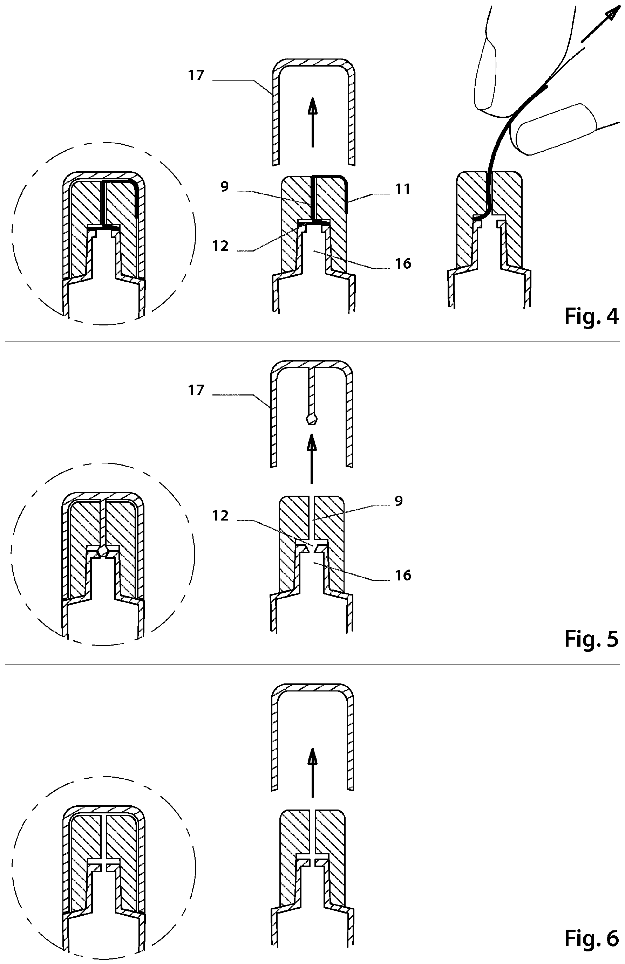

The fluid reservoir may further be provided with an opening means (11) to open said reservoir to provide an aperture (12) to thereby form a fluid communication between the reservoir (10) and the fluid dispensing means (3). Any suitable opening means may be used. The opening means may be a single use opening means or may be resealable to enable reuse of the reservoir. Suitable opening means include pull tab, pull string, foil loop, snap seals, piercer, and pressure burst seal and tear strip. Examples of a resealable opening means include sealed caps such as screw tops; adhesive tabs, hook and loop fasteners such as Velcro™, plastic zips such a Ziploc™, press seals, stopper caps or plugs, valves such as squeeze valves, duck bill valves, push valves and one way valves, and other types of commercially available sealing methods as found on drinks containers for example and know to the skilled person. Figures 4, 5 and 6 show pull tab, stopper caps and cap embodiments.

In one embodiment the reservoir is provided with at least one sealed edge (15) having two surfaces and an opening means (11) is releasably attached in between said two surfaces and extends outwardly there from. Upon removal of said opening means (11) by the user, an opening (12) is formed in the sealed edge (15) to enable the user to dispense the fluid contained in the reservoir (10).

The fluid reservoir (10) may further provided with an opening means tab (11) to open the sealed edge or aperture of the reservoir. The opening means tab (11) may be readily grasped by the consumer, typically at its distal end or by the tag, if present, to initiate the opening process. Typically, the consumer will pull on the opening means tab generally in a direction away from the reservoir and thereby rupture a portion of the sealed edge (15) or aperture to create an opening upon removal or partial removal of the opening means there from. The fluid contained in the reservoir may then be dispensed by the user.

Accordingly, the opening means (11) may be releasably attached in-between the two contacting surfaces which are sealed to provide the at least one sealed edge (15) of the fluid reservoir and extends outwardly there from. The opening means or tab may have a proximal end and a distal end, wherein at least a portion of the proximal end is releasably attached in between the two contacting surfaces of the sealed edge (15) and the distal end extends outwardly there from. Any means may be used to releasably attach the opening means (11) to the two surfaces of the sealed edge (15) or aperture including but not limited to adhesives, heat and pressure sealing, heat sealing being preferred. The opening means (11) is typically positioned in-between the two adjacent surfaces prior to sealing to form the sealed edge (15) as discussed hereinafter. The sealed edge containing the opening means is preferably provided in the top sealed edge of the reservoir.

The opening means may be provided from any suitable material such as the same or different material or film used for the reservoir material as described hereinabove. Suitable materials include but are not limited to metal, cotton, polymers such as polyester, nylon, rayon, plastics, cellulose based materials such as cardboard and paper which may be laminated, coated or waxed. The opening means may be flexible or rigid.

The opening means may have any suitable size, shape and geometry provided that it can be releasable attached in between the two surfaces of the sealed edge and can be easily grabbed by the fingers of consumer. Preferably the opening means is substantially flat. For beauty and grooming applications, the opening means or tab may have a width of from about 0.1mm to 2.5cm, preferably from about 0.5mm to 1cm and a length of from about 1cm to 15cm, preferably from about 2cm to about 10cm. Alternative applications may however require dimensions of from 2cm to 10cm in width and 10cm to 50cm in length. The distal end of the opening means, which extends from the seal and is clearly visible to the consumer, may be symmetrical or unsymmetrical, uniform or non uniform cross section.

The opening means (11) may comprise a single tab, string or thread which extends from said at least one sealed edge or aperture and terminates at a point distal there from. Alternatively, the opening means may be a tab, string or thread which extends from said at least one sealed edge or aperture to form a loop. In such embodiments, the proximal end of the loop may be releasably attached at said at least one sealed edge. The distal end may also be attached in-between the two surfaces of the sealed edge or attached at the exterior surface of the sealed edge or at the front or back surface of the reservoir or attached to a portion of the distal end of the opening means. In one embodiment, both the proximal and distal ends are releasably attached in between the two surfaces of the sealed edge. In one embodiment, the tab may be provided with at least 2, preferably at least 3, more preferably at least 4 tines, in at least a portion of the proximal end of the opening means. The tines may be present in the portion of the proximal end in between the two surfaces forming the sealed edge and may extend into the interior cavity of the reservoir. The tines if present may also extend into a portion of the distal end. The tines may assist in the creation of a more uniform opening or where desirable the creation of more than one opening upon removal of the opening means from the reservoir. Such multiple openings may assist in a more uniform distribution of the fluid upon dispensing from the reservoir onto a surface.

For embodiments where the opening means or tab terminates at a point distal from the sealed opening, the tab may further comprise a tag attached thereto. The tag is preferably substantially wider than the distal end of the opening means or tab to provide a larger surface area for the consumer to grasp and subsequently pull and remove or at least partially detach the opening means from the sealed edge. In an alternative embodiment, the tag may be in the form of a loop attached to the distal end of the opening means to enable the consumer to grasp and pull on the opening means. The tag may be any shape and preferably have a width or diameter of at least 0.1mm, preferably at least 2mm, more preferably at least 5mm, even more preferably from 2mm to 40mm, most preferably from 5mm to 20mm. The width may be uniform or it may be tapered. For embodiments wherein the distal end of the opening means is in the form of a loop, such a tag may be provided at substantially the midpoint of the loop to provide additional assistance to the consumer to grasp the opening means. For embodiments wherein the opening means is a string or thread, the tag may be formed by providing a knot or loop at the distal end of the opening means. The tag may be provided from the same or different material as the opening means. In one embodiment the tag is formed from a different material preferably so as to provide a consumer preferred tactile surface.

The opening means or tab may be provided in an unfolded or folded configuration, which may be held in place by the tab if present and is unfolded prior to use.

The opening means or tab and or tag may be provided with indicia to indicate to the consumer, the location of the tab, and or the preferred gripping location and or the direction to pull the means or tab to open and at least partially detach or remove the tab from the reservoir. Indicia may be in the form of differentiated colours and or symbols.

A portion of the proximal end of the opening means or tab (11) may extend beyond the at least one sealed edge (15) into the interior cavity of the reservoir (10). Such embodiments may further ensure the attachment of the opening means (11) in between the two surfaces of the sealed edge (15). The opening means or tab (11) located in the interior cavity of the reservoir may have the same or different shape as the distal portion of the opening means or tab which extends outward from the reservoir. In one embodiment, the portion of the proximal end which extends into the cavity of the reservoir is in the form of a loop. In such embodiments, the distal end of the opening means extending from the sealed edge may or may not also be in the form of a loop.

The portion of the proximal end of the opening means that extends into the interior cavity of the reservoir may be attached or partially releasably attached to the interior surface of the reservoir.

For embodiments wherein the opening means is a loop, the distal portion may also extend into the reservoir interior.

In one embodiment, the portion of the proximal end of the opening means or tab (11) in the interior cavity of the reservoir has a width which is larger than the portion of the proximal end of the opening means in between the two surfaces at the sealed edge (15). Alternatively, the portion of the proximal end of the opening means in the interior cavity may be larger than the distal portion of the tab extending outward from the sealed edge. Whilst not being bound by theory it is believed that increasing the width of portion of the proximal end of the opening means in the interior cavity of the reservoir results in a greater force being exerted upon the sealed edge as the consumer pulls on the distal end of the opening means. This thereby further improves the opening of the seal upon removal or partial removal of the opening means from the reservoir and optionally also may remove any debris present such as adhesive. The portion of the proximal end of the opening means or tab which extends into the interior cavity of the reservoir may be provided from a different material to that portion in-between the edge seal or the proximal end extending outward there from. The material will be selected to be inert towards the fluid contained within the reservoir. For embodiments wherein the distal end is provided by a string or thread, the width may be increased by the provision of knots or at least one tag.

In another embodiment, the opening means or tab may be tapered in at least a portion, preferably all of the distal and or proximal ends. The opening means may be tapered at least in the portion of the proximal end releasably attached in between the two surfaces of the sealed edge. The opening means may therefore have a width in a portion of the proximal or distal end that is larger than the portion of the proximal end of the opening means positioned between the two surfaces forming the sealed opening. Similarly, a portion of the proximal end of the opening means may have a width which is larger than the width of the portion of the proximal end positioned between the two surfaces of the sealed edge. The tapering may be linear or curved so as to provide an hour glass or arrow head shape for example. This is of particular advantage for embodiments wherein the proximal end extends into the cavity of the reservoir. Upon exertion of force to remove the opening means the portion of the proximal end thereof located in the reservoir cavity having a width larger than the width of the portion of the proximal end located in between the two surfaces of the sealed edge will be forced against the sealed edge and cause the seal to be ruptured, thereby creating an opening and or enlarging the opening(s).

The opening tab may be positioned at any position along the at least one sealed edge. Preferably the opening tab is located at substantially the midpoint of said sealed edge, but may be located at a position to the left or right of the midpoint or at or towards the corner of the sealed edge.

In one embodiment wherein the opening means or tab is provided in the form of a loop wherein the distal and proximal ends are releasably attached in between the two surfaces of the sealed edge, the opening means and reservoir are preferably provided from the same or substantially similar material as the reservoir. Such material may preferably be provided with different properties for each surface which may be readily provided by laminate materials to enable heat sealing. The outer surface of loop laminate which contacts the inner surface of the sealed edge is selected such that it will weakly adhere to the surface of the laminate film of the reservoir at the sealed edge. Whilst not being bound by theory it is believed that this results in a seal that requires less force to be opened upon removal or partial removal of the opening means upon the application of force. This is particularly advantageous for multi-layered composite films, for example (PET/VMPE) and other commercially available films.

In addition to releasably attaching a portion of the proximal end of the opening means in between the two surfaces of the sealed edge (15) of the reservoir (10), a portion of the distal or proximal end of the opening means may have at least one additional attachment, preferably a releasable attachment to the reservoir. Such an attachment may be on an external or internal surface of the reservoir. Embodiments wherein the opening means and or the reservoir utilize laminate materials utilizing adhesives in their manufacture this may find particular utility to form such an attachment. Whilst not bound by theory it is believed that the adhesive may seep from the laminate particularly due to the application of heat and or pressure, around the perimeter sealed edges and or edges of the opening means and thereby result in additional attachment.

### Neck portion

In one preferred embodiment, the reservoir (10) and or handle (4) if the handle is providing a hollow cavity acting as the fluid reservoir comprises a main body which is further provided with a neck portion (16) extending there from. The presence of a neck portion (16) enables improved fluid flow control and may also enable improved connection with the device to which the reservoir may be attached. The at least one sealed edge (15) or aperture may be located in the body portion of the reservoir or handle or it may be located in the neck portion (16). Preferably the at least sealed edge is located in the neck portion, if present. The top and side edges of the reservoir if present will extend from the body to neck portion respectively.

The neck portion is preferably located substantially at the midpoint of the width of the body portion extending from the top edge thereof. Alternative configurations include embodiments where the neck portion is offset form the midpoint or located towards one of the upper corner edges of the body portion. The neck portion may extend a length of up to 50% of the length of the side edge of the body portion. In such embodiments the side edges of the reservoir may extend from the body potion to form the side edges of the neck portion and similarly the top edge or a portion of the top edge of the reservoir may be located in the neck portion. Preferably the neck portion has a width less than the width of the body of the reservoir, preferably less than 75%, preferably less than 50%, more preferably less than 40% of the width of the body portion of the major front or rear surface of the reservoir

The neck portion may have any suitable shape and may be symmetrical or unsymmetrical and is preferably selected to enhance the flow of fluid towards the opening. The neck portion may have substantially linear sides or curved sides which may be substantially vertical or at a gradient to provide a tapered neck which aids in the funneling of the fluid out of the reservoir.

In a preferred embodiment the opening means extends from the reservoir through the handle (4) and or fluid dispensing means (3) at least to the front surface of the device so that the distal end or tag can be readily accessed by the consumer as shown in Fig 4. In such embodiments the fluid dispensing means (3) may be provided with at least one aperture or opening (9) sized to enable the opening means (11) to be passed through the fluid dispensing means (3). Such opening (9) may also function to deliver the fluid to the skin form the reservoir (10).

For embodiments wherein the fluid reservoir is preferably provided with a neck portion, the neck portion may extends towards the fluid dispensing means and even more preferably extends at least a portion within or onto or through the fluid dispensing means via an opening or aperture and thereby create a fluid pathway between the fluid reservoir and the fluid dispensing means. The opening means is typically located in the neck portion if present.

The fluid dispensing means has at least one fluid pathway in fluid communication with said opening means and said reservoir. For embodiments wherein the handle is positioned between the fluid reservoir and the fluid dispensing means, the handle may be provided with an opening through which a portion of the reservoir at least partially extends through. Alternatively the fluid reservoir may extend around the handle to access the fluid dispensing means.

### Cap/cover

The hair removal and fluid application device may further comprise a cap(s) and or cover (s) and or disposable seal or release liner to protect the hair removal means and or fluid dispensing means prior to use, during use and after use for multiple use embodiments. Any suitable cap (17) or seal may be used such as flow wraps. The cover may in addition completely cover and enclose the device. The cap may be provided in the same material as described for the handle or a different material. The cap or cover may be integrated with the handle or separate therefrom as shown in the figures

### Fluid

The reservoir comprises a liquid, gel or paste which may comprise skin and or shaving care actives and or hair removal or depilatory compositions. The compositions may be aqueous, water in oil or oil in water emulsions.

It has been found that when selecting a composition to be used in hair removal devices, it can be particularly desirable to select a composition which is sufficiently thick and viscous that it will not run off the skin or razor after being dispensed. Additionally, moisturizing compositions can be desirable for use in a fluid dispensing hair removal device to allow for multiple benefits, including but not limited to hydration of the hairs prior to shaving, moisturization of skin during the hair removal process, lubrication of skin to reduce friction during the shave, and so forth. Those of skill in the art will understand that moisturization can include hydration of the skin or hair or occlusion of the skin and or hair, or lubrication of the hair or skin to increase glide and reduce friction between the fluid dispensing device and skin.

### Water

The shave care composition of the current invention comprises water. In one embodiment, the shave care composition comprises at least about 30% by weight water. In an alternate embodiment, the shave care composition comprises at least about 40% by weight water. In an alternate embodiment, the shave care composition comprises at least about 50%, more preferably at least 60%, even more preferably at least 80% and even more preferably at least 90% by weight water. Compositions having high levels of water enable the device to be used without the necessity for an additional water source to apply or remove the composition from the skin after application.

### Lipophilic Skin Conditioning Agent

Shave care composition of the present invention may comprise one or more lipophilic skin conditioning agents. The concentration level of the skin conditioning agents either singularly or collectively may range from about 1% to about 50% by weight of the base composition, preferably about 10% to about 40%, and more preferably from about 13% to about 30%. Exemplary skin conditioning agents include hydrocarbons, polymeric hydrocarbons, esters, ethers, and silicones selected from the group consisting of alkyl ethers, mineral oil, isoparaffin, greater than C20 hydrogenated polyisobutene; and an ester composed of a branched C16-C22 alkyl chain and a mono alkyl group consisting of a linear or branched C1 to C6 alkyl chain. Some preferred skin conditioning agents comprise isostearic acid derivatives; for example, isostearyl isostearate, isopropyl isostearate, isopropylpalmitate, isopropylmyristate, PPG-15 Stearyl Ether, petrolatum, dimethicone and dimethoconol and mixtures thereof.

In one embodiment, two or more hydrocarbon phases are preblended prior to emulsification. It has been found that pre-blends of such ingredients can lead to improved skin feel. Examples include petrolatum blended with mineral oil or isopropylpalmitate.

The skin conditioning agents may also help to reduce the coefficient of friction for compositions provided herein. The reduction in friction can decrease the potential for skin irritation that can arise from contacting the skin one or more times with a hair removal device such as a razor blade. Employment of the skin conditioning agent in this context may also permit formulation flexibility regarding the type and concentration level of lubricants that are included in the shaving preparations.

In one embodiment of the invention, particle size of the dispersed phase skin conditioners has an average particle size of 95% of the dispersed phase mass below 20 microns, preferably below 15 microns, more preferably below 10 microns and most preferably below 5 microns. Particle size as measured using a Horiba particle size analyzer and reported as D 50 values. While not wishing to be bound by theory, the smaller particle size is very important for the dispersed phase skin conditioners to be retained on the skin during shaving especially when the shaving composition is dispensed in front of the razor blades or upon re-stroke of the razor when the composition has been deposited on the skin. It is recognized that the skin is not a flat surface and smaller particles can deposit and reside in the recessed areas of the skin and around the hair follicle more easily than larger particles.

### Thickening Agent

The fluid composition may contain one or more thickening agents, from about 0.1% to about 5%, alternatively from about 0.1% to about 4%, alternatively from about 0.25% to about 3%, by weight of the composition.

Non limiting classes of thickening agents include those selected from the following: Carboxylic Acid Polymers, Crosslinked Polyacrylate Polymers Polyacrylamide Polymers, Polysaccharides, Clays and Gums, and mixtures thereof when appropriate. In one embodiment, compositions of the present invention include a thickening agent selected from carboxylic acid polymers, crosslinked polyacrylate polymers, polyacrylamide polymers, polysaccharides, and mixtures thereof, more preferably selected from carboxylic acid polymers, polyacrylamide polymers, polysaccharides, and mixtures thereof.

Preferred thickening/suspending agents include electrolyte sensitive polymers that are shear thinning when in solution. Shear thinning is property that makes a liquid easy to spread and pump. We have found that electrolyte sensitive polymers have desired performance profiles. While not wishing to be bound by theory, the electrolyte sensitive polymers interact with the residual surfactant or electrolyte left on the skin and release the lubrication agents and/or suspended conditioning agents for spreading across the razor and across the surface of the skin. Preferred electrolyte sensitive polymers include but are not limited to: Polyacrylamide, Hydroxyethyl Acrylate/Sodium Acryloyldimethyltaurate Copolymer, Sodium Acrylate/Sodium Acryloyldimethyl Taurate Copolymer, Ammonium Polyacrylate, Sodium Acrylate/Acryloyldimethyltaurate/Dimethylacrylamide Crosspolymer, Hydroxyethyl Acrylate/Sodium Acryloyldimethyltaurate Copolymer which can be purchased from Seppic or Carboxylic Acid Polymers (Carbomers) such as Ultrez 10, Carbopol 934, Carbopol 980 and ETD 2050 which can be purchased from Lubrizol or Ammonium Acryloyldimethyltaurate/VP Copolymer, Sodium Acryloyldimethyltaurate/VP Copolymer, Ammonium Acryloyldimethyltaurate/Beheneth-25 Methacrylate Crosspolymer, which can be purchased from Clariant. The most preferred electrolyte sensitive polymer is Polyacrylamide available as Sepigel 305 (Polyacrylamide & C13-14 Isoparaffin & Laureth-7).

### Emulsifier

The fluid composition may contain one or more emulsifying agents, from about 0.1% to about 20%, alternatively from about 0.5% to about 15%, alternatively from about 1.0 % to about 12%, by weight of the composition. Non limiting examples of surfactants for emulsification for use herein are disclosed in McCutcheon's, Detergents and Emulsifiers, North American edition (1986), published by allured Publishing Corporation; and McCutcheon's, Functional Materials, North American Edition (1992). Preferred emulsifiers are nonionic surfactants/emulsifiers. Non limiting useful emulsifiers herein include those selected from the group consisting of alkyl glucosides, alkyl polyglucosides, polyhydroxy fatty acid amides, alkoxylated fatty acid esters, sucrose esters, alkoxylated fatty alcohols, amine oxides, and mixtures thereof. Most preferred are alkoxylated fatty alcohols and alkyl glucosides and mixtures thereof.

In one embodiment the fluid composition comprises less than about 5%, or less than about 3%, or less than about 2% of one or more lathering surfactants. In one embodiment the fluid is free or substantially free of lathering surfactants. A lathering surfactant is defined as a surfactant which when combined with water and mechanically agitated generate a foam or later. Lathering surfactants include anionic and amphoteric lathering surfactants and mixtures thereof. Anionic lathering surfactants include sarcosinates, sulfates, sulfonate, isethionate, taurates, phosphates, lactylates, glutamates, alkali metal salts of fatty acids (i.e. soaps) having from 8 to 24 carbons, and mixtures thereof.

### Lubricants

The fluid compositions may employ one or more lubricants, from about 0.1% to about 8%, alternatively from about 0.1% to about 5%, alternatively from about 0.2% to about 3%, by weight of the composition. Exemplary lubricants include lubricous water soluble polymers, water insoluble particles, and hydrogel-forming (or water swellable) polymers, and mixtures thereof.

Useful lubricious water soluble polymers may have a molecular weight greater between about 300,000 and 15,000,000 daltons, preferably more than about one million Daltons. Nonlimiting examples of suitable lubricious water soluble polymers include polyethylene oxide, polyvinylpyrrolidone, and polyacrylamide. Non limiting useful water insoluble particles may include inorganic particles or organic polymer particles. Hydrogel-forming polymers are typically highly hydrophilic polymers that, in water, form organized three-dimensional domains of approximately nanometer scale. Additional polymer lubricants include: cellulose derivatives such hydroxyalkyl cellulose polymers such as hydroxyethyl cellulose and hydroxypropyl cellulose, carboxymethyl cellulose, and cellulose methyl ether and polysaccharide gums such as, for example, xanthan gum, carrageenan gum, guar gum, locust bean gum, and hydroxypropyl guar gum.

### Sensates

In one embodiment of the invention, the composition may contain sensates, or combinations of sensates. Sensates can be materials that provide the sensation of a thermal change, e.g., heating or cooling. Applicants have found that the addition of sensates using this composition provides longer lasting skin sensation and comfort benefits. Non-limiting examples include: p-Methane-3,8-diol; Isopulegol; Menthoxypropane-1,2,-diol; Curcumin; Menthyl Lactate; Gingerol; Icilin; Menthol; Tea Tree Oil; Methyl Salicylate; Camphor; Peppermint Oil; N-Ethyl-p-menthane-3-carboxamide; N-[4-(Cyanomethyl)phenyl]-2-isopropyl-5-methylcyclohexane-carboxamide; Ethyl 3-(p-menthane-3-carboxamido)acetate; 2-Isopropyl-N,2,3-trimethylbutyramide; Menthone glycerol ketal, and mixtures thereof.

### Gel Network

The fluid composition is preferably substantially free from a gel network phase. As used herein, the term "gel network" refers to a lamellar or vesicular solid crystalline phase which comprises at least one fatty amphiphile. The present invention contains less than about 3%, alternatively less than about 1%, alternatively less than about 0.5% of at least one fatty amphiphiles. Gel networks have been found to reduce the rinse profile of these systems. Fatty alcohol gel networks have been used for years in cosmetic creams and hair conditioners. Gel networks are a re-solidified liquid crystal gel phase formed by fatty amphiphiles (e.g. cetyl or stearyl alcohol) and a hydrophilic phase (e.g. water). It is formed by undergoing a melting and then re-solidification process in the hydrophilic phase. The gel network will typically have a lower thermal transition than the melt temperature of the fatty amphiphile itself.

### Optional Ingredients

The fluid composition may further comprise additional optional ingredients. Suitable additional optional ingredients include perfume, preservatives, chelants, sensates (e.g. menthol), desquamation actives, anti-acne actives, anti-wrinkle/anti-atrophy actives, anti-oxidants/radical scavengers, flavonoids, anti-inflammatory agents, anti-cellulite agents, topical anesthetics, tanning actives, skin lightening agents, skin soothing and healing actives, antimicrobial actives, sunscreen actives, visual skin enhancers, humectants and moisturizing agents (e.g., glycerin, glycols, sorbitol) and the like. Such optional ingredients are described more fully in U.S. Application Serial No. 11/367,918, filed March 3, 2006. Preferred additional optional ingredients include salicylic acid, opacifiers (e.g. mica and titanium dioxide), perfume, hydrophilic conditioning agents (e.g., glycerin) and skin sensates (e.g. menthol).

The fluid composition may contain salicylic acid, its isomers, tautomers, salts and derivatives thereof. Alternatively, the compositions comprise from about 0.001% to about 5% salicylic acid. Alternatively, the compositions comprise from about 0.01% to about 2% salicylic acid. Alternatively, the compositions comprise from about 0.1% to about 1% salicylic acid. Without wishing to be bound by theory, it is believed that salicylic acid is efficacious for the treatment of acne on the skin. Moreover, the salicylic acid is capable of treating and/or reducing the presence of acne on the skin. Such treatment with the shave care composition of this invention involves applying the shave care composition to the skin via the razor and shaving the skin that has been treated with the shave care composition.

Derivatives of salicylic acid include, but are not limited to, any compounds wherein the CH3 groups are individually or in combination replaced by amides, esters, amino groups, alkyls, and alcohol esters. Tautomers of salicylic acid are the isomers of salicylic acid which can change into one another with ease so that they ordinarily exist in equilibrium. Thus, tautomers of salicylic acid can be described as having the chemical formula C7H6O3 and generally having a similar structure to salicylic acid.

The compositions of the present invention may include from about 0.001% to about 5%, alternatively from about 0.01% to about 2%, and alternatively from about 0.1% to about 1%, of alpha- or beta-hydroxy acids, and derivatives, salts, isomers and tautomers thereof. Non-limiting examples of alpha- and beta-hydroxy acids include alpha-hydroxy-butyric acid, alpha-hydroxyisobutyric acid, alpha-hydroxyisocaproic acid, alpha-hydroxyisovaleric, atrolactic acid, beta-hydroxybutyric acid, beta-phenyl lactic acid, beta-phenylpyruvic acid, citric acid ethyl pyruvate, galacturonic acid, glucoheptonic acid, glucoheptono 1,4-lactone, gluconic acid, gluconolactone glucuronic acid, glucuronolactone, glycolic acid, isopropyl pyruvate, lactic acid, malic acid, amndelic acid, emthyl pyruvate, mucic acid, pyruvic acid, saccharic acid, saccharic acid 1,4-lactone, tartaric acid and tartronic acid, and mixtures thereof.

Opacifiers may be added to the shave care composition of the present invention. Opacifiers may be either inorganic or organic compounds. Inorganic opacifiers include, for example, titanium dioxide, zinc oxide, talc, mica or coated mica (with oxides of titanium, tin, or iron or bismuth oxychloride), magnesium aluminum silicate, bismuth oxychloride, or other minerals. These compounds can be added as powders, dispersions, or complexes. Organic opacifiers include, for example, opaque emulsions (e.g., containing Styrene/PVP copolymer, vinyl polymers, or latexes), metal salts of amines containing 14-20 carbon atoms per molecule, alkanolamides containing 14-20 carbon atoms per molecule, organic alcohols containing 14-20 carbon atoms per molecule, insoluble salts of stearic acid, glycol mono-or distearates, propylene glycol and glycerol monostearates and palmitates. Combinations of these opacifiers can also be used. The opacifying additive is typically included in an amount of about 1 to about 6%, preferably about 2 to about 5%, by weight of the composition.

The fluid compositions may include depilatory actives including any keratin reducing agents such as sulphide salts, thioglycol, thioglycerol, thioglycomide, thioglycolhydrazide thioglycolic acid, thioglycolate salts such as potassium, calcium and ammonium, thiosalicylic acid, thiomalic acid, ammonium thiolactic acid, cysteine and cysteamine. The reducing agent is present at amounts of from about 0.1% to 20%, preferably 0.2% to 15%, more preferably from 0.5% to 10% by weight of the composition. Preferably the depilatory composition may further comprise a base to control pH such as sodium or potassium hydroxide, ammonia alkanolamides such as monoethanolamide and mixtures thereof.

### Specific embodiments

The invention will now be further descried with reference to specific embodiments of the invention.

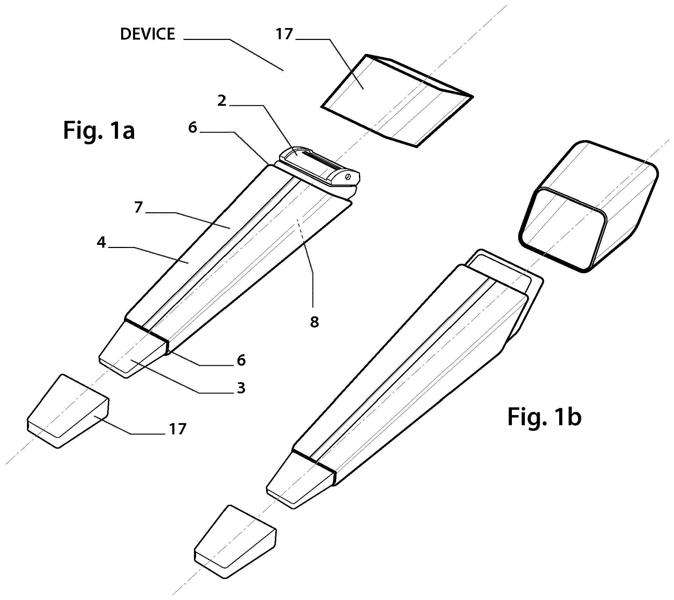

Figures 1 a and b show a first embodiment of the invention. This embodiment comprises a hair removal means (2) comprising at least one razor blade provided in a housing located on the proximal end of the handle on the front surface of the handle (4) . A fluid dispensing means (3) is located on the distal end of the handle (4) whereby the skin contacting surface of the hair removal means (2) and fluid dispensing means (3) are positioned in substantially opposing directions. The hair removal means (2) and the fluid dispensing means (3) are arranged in a predetermined position such that they are located in opposing directions facing the respective skin contacting surfaces. The fluid dispensing means (3) comprises a sponge which extends substantially across the entire width of the distal end 5 of the handle (4). The hair removal means extends substantially across the entire width of the proximal end (6) of the handle. The hair removal means (2) and fluid dispensing means (3) are secured to the handle (4). The handle (4) is formed from a single piece of carton board which may be coated with a water repellant material or laminated. The carton board is formed into a tubular shape and adhered together and the fluid reservoir (10) which comprises a laminated sheet is located therein so that the handle (4) substantially covers the front and back surfaces of the fluid reservoir (10) which is retained therein. The handle (4) is provided with an opening through which a portion of the reservoir is inserted to form a liquid pathway to the fluid dispensing means (3). The hair removal means (2) and fluid dispensing means (3) are provided with protective caps (17).

Figures 2a and b show a second and third embodiment of the invention. Both embodiments comprise a hair removal means (2) comprising at least one razor blade provided in a housing located on the proximal end (5) of the handle and a fluid dispensing means (3) located on the distal end (6) of the handle whereby the skin contacting surface of the hair removal means and fluid dispensing means are positioned in substantially opposing directions. The hair removal means (2) and the fluid dispensing means (3) are arranged in a predetermined position such that they are located in opposing direction facing the respective skin contacting surfaces. The fluid dispensing means (3) comprises a sponge/felt which extends substantially across the entire width of the distal end (6) of the handle (4. The handle (4) provides a hollow cavity which provides the fluid reservoir. The handle has a neck (16) which provides the sealed aperture (12) and which is provided with an opening means (11) which upon opening forms a fluid communication between the reservoir and the fluid dispensing means. The fluid dispensing means is provided with a protective cap (17). The hair removal means extends substantially across the entire width of the proximal end (5) of the handle (4) and is provided with a protective cap (17). The hair removal means (2) and fluid dispensing means (3) are secured to the handle (4). The hair removal means is mounted on the proximal end (5) of the handle (4) which is inclined away from the vertical plane.

In the third embodiment shown in figure 2b, the inclined proximal end (5) of the handle is provided with a living hinge (19) to enable the hair removal means to pivot.

Figures 3a and 3b show a fourth embodiment of the invention. This embodiment comprises a hair removal means (2) comprising at least one razor blade provided in a housing located on the proximal end (5) of the handle on the front surface of the handle (4). A fluid dispensing means (3) is located on the distal end (6) of the handle whereby the skin contacting surface of the hair removal means and fluid dispensing means are positioned in substantially opposing directions. The hair removal means (2) and the fluid dispensing means (3) are arranged in a predetermined position such that they are located in opposing directions facing the respective skin contacting surfaces. The fluid dispensing means (3) comprises a sponge which extends substantially across the entire width of the distal end (6) of the handle (4). The hair removal means extends substantially across the entire width of the proximal end (6) of the handle. The hair removal means (2) and fluid dispensing means (3) are secured to a handle (4). The handle (4) provides a hollow cavity which provides the fluid reservoir (10). The handle has a neck (16) which provides the sealed aperture (12) and which is provided with an opening means (11) which upon opening forms a fluid communication between the reservoir and the fluid dispensing means (3). The fluid dispensing means is provided with a protective cap (17). The proximal end (5) of the handle is provided with a chassis (13) with a pivot onto which the hair removal means (2) is mounted. The hair removal means (2) is also provided with a protective cap (17).

Figure 4 shows a partial schematic cross section of the handle (4) providing a hollow cavity reservoir (10) having a neck portion (16) and a sealed aperture (12) connected to a pull tab opening means (11) and the opening thereof.

Figure 5 shows a partial schematic cross section of the handle (4) providing a hollow cavity reservoir having a neck portion (16) sealed by a stopper attached to a protective cap (17).

Figure 6 shows a partial schematic cross section of the handle (4) wherein the aperture (12) to the reservoir is sealed by the protective cap (17).

Figures 7a, 7b and 7c show an eighth embodiment of the invention, This embodiment comprises a hair removal means (2) comprising at least one razor blade provided in a housing located on the proximal end (5) of the handle on the front surface of the handle (4). A fluid dispensing means (3) is located on the distal end (6) of the handle whereby the skin contacting surface of the hair removal means and fluid dispensing means are positioned in substantially opposing directions. The hair removal means (2) and the fluid dispensing means (3) are arranged in a predetermined position such that they are located in opposing directions facing the respective skin contacting surfaces. The fluid dispensing means (3) comprises a felt nib. The hair removal means extends substantially across the entire width of the proximal end of the handle. The hair removal means (2) and fluid dispensing means (3) are secured to a handle (4). The handle is provided with a reservoir (10) to contain the fluid. At the distal end (6) of the handle the reservoir is provided with a plug (18) which provides an aperture (12) from the reservoir and forms a channel through the felt nib. The fluid dispensing means is sealed by a protective cap (17). The proximal end (5) of the handle is provided with a chassis (13) with a pivot onto which the hair removal means (2) is mounted. The hair removal means is also provided with a protective cap (17).

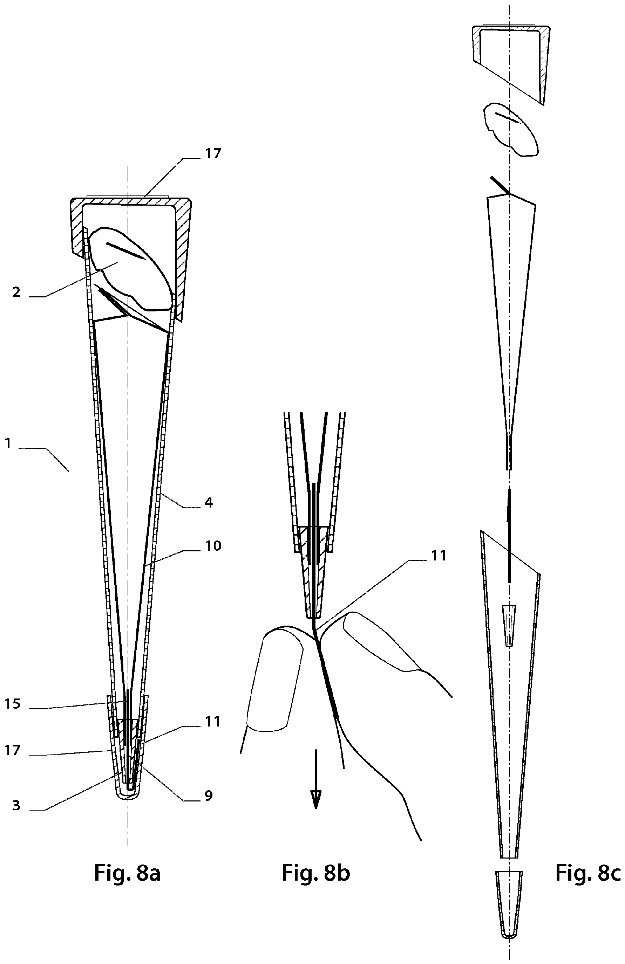

Figures 8a, 8b and 8c show a ninth embodiment of the invention which is an alternative of the eighth embodiment. This embodiment comprises a hair removal means (2) comprising at least one razor blade provided in a housing located on the proximal end (5) of the handle on the front surface of the handle (4). A fluid dispensing means (3) is located on the distal end (6) of the handle whereby the skin contacting surface of the hair removal means and fluid dispensing means are positioned in substantially opposing directions. The hair removal means (2) and the fluid dispensing means (3) are arranged in a predetermined position such that they are located in opposing directions facing the respective skin contacting surfaces. The fluid dispensing means (3) comprises a felt nib. The hair removal means extends substantially across the entire width of the proximal end of the handle. The hair removal means (2) and fluid dispensing means (3) are secured to a handle (4). The sealed edges (15) of the reservoir extend into the felt nib and are provided with a pull tab opening means (11) which extends through the felt nib. The fluid dispensing means is sealed by a protective cap (17). The hair removal means is also provided with a protective cap (17).

### Method of Use

The hair removal and fluid dispensing devices of the invention provide a simple and convenient method to effectively remove hair from the body. The consumer typically, removes any cap or release layer present to protect the hair removal means or fluid dispensing means. The consumer then opens the fluid reservoir and removes the opening means there from. Optionally the consumer may apply pressure against the fluid reservoir to facilitate the flow of fluid from the reservoir to the fluid dispensing means. The consumer then places the fluid dispensing means on the skin surface to be treated and applies the fluid using the dispensing means to the surface to be treated. The device is then rotated and the consumer applies the hair removal device to the surface to remove the hair. The process is then repeated on the same or additional surfaces until the desired hair removal is achieved. In addition the consumer may position and apply the fluid dispensing means over the same surface after the hair removal step in order to collect and remove any debris or excess composition from the skin surface or to apply fluid to the skin surface. Alternatively, the consumer may utilize the hair removal means first before the fluid dispensing means, which may be used to deliver after shave composition. The hair removal and fluid dispensing device of the present invention does not require a source of water in order to function, in particular the skin does not require wetting or the application of any pre and or post shave composition. Moreover the device does not require any post use rinsing.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A removal and fluid application device (1) having a hair removal means (2), a fluid dispensing means (3), a handle and a fluid reservoir (10) contained therein; wherein said fluid dispensing means (3) is attached to the distal end (6) of said handle (4) and said hair removal means (2) is attached to the proximal end (5) of said handle (4),
wherein said handle (4) provides a predetermined location of said hair removal means (2) and said fluid dispensing means (3) to one another, **characterized in that** the handle (4) is substantially rigid and wherein said dispensing means (3) is an open cell foam or a closed cell foam,
wherein said reservoir (10) is sealed and further comprises an opening means (11).

2. A hair removal and fluid application device (1) according to claim 1, wherein said device comprises a front surface (7) and a back surface (8) wherein said front surface (7) comprises said hair removal means (2).

3. A hair removal and fluid application device (1) according to claim 2, wherein said back surface comprises said fluid dispensing means (3).

4. A hair removal and fluid application device (1) according to claim 1, wherein said handle (4) is substantially linear.

5. A hair removal and fluid application device according to claim 1, wherein said opening means (11) comprises a tab, thread or string.

6. A hair removal and fluid application device (1) according to any one of the preceding claims, wherein said hair removal means (2) comprises at least one blade; preferably a blade and a housing.

7. A hair removal and fluid application device (1) according to any one of the preceding claims, wherein said handle (4) at least partially, preferably substantially encloses said fluid reservoir (10).

8. A hair removal and fluid application device (1) according to any one of the preceding claims, wherein said reservoir (10) is in fluid communication with said fluid dispensing means (3).

9. A hair removal and fluid application device according to any one of the preceding claims, wherein said fluid dispensing means (3) has at least one aperture in fluid communication with said reservoir (10).

10. A hair removal and fluid application device (1) according to any one of the preceding claims, wherein said reservoir (10) comprises a composition comprising at least 80% by weight water and from 0.1% to 5% by weight of a thickening agent.

11. A method to apply fluid and remove hair from the skin comprising the steps of contacting a skin surface with the hair removal means (2) or fluid dispensing means (3) of a device (1) according to claim 1, preferably in the absence of a source of water.

## Patentansprüche

1. Entfernungs- und Fluidauftragungsvorrichtung (1) mit einem Haarentfernungsmittel (2), einem Fluidabgabemittel (3), einem Griff und einem darin enthaltenen Flüssigkeitsbehälter (10); wobei das Fluidabgabemittel (3) an dem distalen Ende (6) des Griffs (4) gebunden ist und das Haarentfernungsmittel (2) an dem proximalen Ende (5) des Griffs (4) gebunden ist,
wobei der Griff (4) eine vorher festgelegte Position des Haarentfernungsmittels (2) und des Fluidabgabemittels (3) zueinander bereitstellt, **dadurch gekennzeichnet, dass** der Griff (4) im Wesentlichen steif ist und wobei das Abgabemittel (3) ein offenzelliger Schaum oder geschlossenzelliger Schaum ist,
wobei der Behälter (10) abgedichtet ist und ferner ein Öffnungsmittel (11) umfasst.

2. Haarentfernungs- und Fluidauftragungsvorrichtung (1) nach Anspruch 1, wobei die Vorrichtung eine Vorderseite (7) und eine Rückseite (8) umfasst, wobei die Vorderseite (7) das Haarentfernungsmittel (2) umfasst.

3. Haarentfernungs- und Fluidauftragungsvorrichtung (1) nach Anspruch 2, wobei die Rückseite das Fluidabgabemittel (3) umfasst.

4. Haarentfernungs- und Fluidauftragungsvorrichtung (1) nach Anspruch 1, wobei der Griff (4) im Wesentlichen linear ist.

5. Haarentfernungs- und Fluidauftragungsvorrichtung nach Anspruch 1, wobei das Öffnungsmittel (11) eine Lasche, einen Faden oder eine Schnur umfasst.

6. Haarentfernungs- und Fluidauftragungsvorrichtung (1) nach einem der vorstehenden Ansprüche, wobei das Haarentfernungsmittel (2) mindestens eine Klinge; vorzugsweise eine Klinge und ein Gehäuse umfasst.

7. Haarentfernungs- und Fluidauftragungsvorrichtung (1) nach einem der vorstehenden Ansprüche, wobei der Griff (4) vorzugsweise im Wesentlichen den Flüssigkeitsbehälter (10) wenigstens teilweise umschließt.

8. Haarentfernungs- und Fluidauftragungsvorrichtung (1) nach einem der vorstehenden Ansprüche, wobei der Behälter (10) in Fluidaustausch mit dem Fluidabgabemittel (3) steht.

9. Haarentfernungs- und Fluidauftragungsvorrichtung nach einem der vorstehenden Ansprüche, wobei das Fluidabgabemittel (3) mindestens eine Öffnung in Fluidaustausch mit dem Behälter (10) aufweist.

10. Haarentfernungs- und Fluidauftragungsvorrichtung (1) nach einem der vorstehenden Ansprüche, wobei der Behälter (10) eine Zusammensetzung umfasst, die mindestens zu 80 Gew.-% Wasser und zu 0,1 bis 5 Gew.-% eines Verdickungsmittels umfasst.

11. Verfahren zum Auftragen von Fluid und Entfernen von Haar von der Haut, umfassend die Schritte des Inkontaktbringens einer Hautoberfläche mit dem Haarentfernungsmittel (2) oder Fluidabgabemittel (3) einer Vorrichtung (1) nach Anspruch 1, vorzugsweise in Abwesenheit einer Wasserquelle.

## Revendications

1. Dispositif d'épilation et d'application de fluide (1) ayant un moyen d'épilation (2), un moyen de distribution de fluide (3), un manche et un réservoir de fluide (10) contenu en son sein ; dans lequel ledit moyen de distribution de fluide (3) est fixé à l'extrémité distale (6) dudit manche (4) et ledit moyen d'épilation (2) est fixé à l'extrémité proximale (5) dudit manche (4),
dans lequel ledit manche (4) fournit un emplacement prédéterminé dudit moyen d'épilation (2) et dudit moyen de distribution de fluide (3) l'un à l'autre, **caractérisé en ce que** le manche (4) est sensiblement rigide et dans lequel ledit moyen de distribution (3) est une mousse à alvéoles ouvertes ou une mousse à alvéoles fermées,
dans lequel ledit réservoir (10) est scellé et comprend en outre un moyen d'ouverture (11).

2. Dispositif d'épilation et d'application de fluide (1) selon la revendication 1, dans lequel ledit dispositif comprend une surface avant (7) et une surface arrière (8) dans lequel ladite surface avant (7) comprend ledit moyen d'épilation (2).

3. Dispositif d'épilation et d'application de fluide (1) selon la revendication 2, dans lequel ladite surface arrière comprend ledit moyen de distribution de fluide (3).

4. Dispositif d'épilation et d'application de fluide (1) selon la revendication 1, dans lequel ledit manche (4) est sensiblement linéaire.

5. Dispositif d'épilation et d'application de fluide selon la revendication 1, dans lequel ledit moyen d'ouverture (11) comprend une languette, un fil ou une ficelle.

6. Dispositif d'épilation et d'application de fluide (1) selon l'une quelconque des revendications précédentes, dans lequel ledit moyen d'épilation (2) comprend au moins une lame ; de préférence une lame et un logement.

7. Dispositif d'épilation et d'application de fluide (1) selon l'une quelconque des revendications précédentes, dans lequel ledit manche (4) enferme au moins partiellement, de préférence essentiellement ledit réservoir de fluide (10).

8. Dispositif d'épilation et d'application de fluide (1) selon l'une quelconque des revendications précédentes, dans lequel ledit réservoir (10) est en communication fluidique avec ledit moyen de distribution de fluide (3).

9. Dispositif d'épilation et d'application de fluide selon l'une quelconque des revendications précédentes, dans lequel ledit moyen de distribution de fluide (3) a au moins une ouverture en communication fluidique avec ledit réservoir (10).

10. Dispositif d'épilation et d'application de fluide (1) selon l'une quelconque des revendications précédentes, dans lequel ledit réservoir (10) comprend une composition comprenant au moins 80 % en poids d'eau et de 0,1 % à 5 % en poids d'un agent épaississant.

11. Procédé pour appliquer un fluide et épiler la peau comprenant les étapes de mise en contact d'une surface de la peau avec le moyen d'épilation (2) ou le moyen de distribution de fluide (3) d'un dispositif (1) selon la revendication 1, de préférence en l'absence d'une source d'eau.
